# EUROPEAN PATENT SPECIFICATION

(11) **EP 0 920 329 B1**
(45) Date of publication and mention of the grant of the patent: **25.09.2002**
(21) Application number: 97919596.3
(22) Date of filing: 09.05.1997
(51) Int. Cl.: A61K 38/21, A61K 9/20

(54) **STABILIZATION OF INTERFERONS IN AQUEOUS SOLUTION BY ARABIC GUM**
STABILISIERUNG VON INTERFERONEN IN WÄSSRIGER LÖSUNG DURCH GUMMIARABIKUM
STABILISATION DES INTERFERONS DANS UNE SOLUTION AQUEUSE PAR DE LA GOMME ARABIQUE

(30) Priority: 09.05.1996 WO PCT/IB96/00433
(43) Date of publication of application: 09.06.1999
(73) Proprietor: FERONPATENT LIMITED, Gibraltar (GI)
(72) Inventor: ROTHSCHILD, Peter R., Cd. Guadalupe, Nuevo León 67100 (MX)
(74) Representative: Moinas, Michel
(86) International application number: IB9700531
(87) International publication number: WO97041885

(56) References cited:
- EP-A- 0 306 423
- EP-A- 0 420 049
- WO-A-88/03411
- CH-A- 156 609
- US-A- 5 298 261
- G. NETIEN ET AL.: "GALENICA", VOL. 16, "MEDICAMENTS HOM OPATHIQUES" 2nd. EDITION" 1986 , TECHNIQUE ET DOCUMENTATION , PARIS, FR XP002022482 see page 91 - page 99

## Description

The invention concerns the stabilisation of interferons in aqueous solution for application in the manufacturing of sublingually administered tablets by homeopathic dilution method for the preventing and treating of interferon sensitive viral infections in humans and for functioning as an immunomodulator.

Interferons belong to a group of glycoprotein and proteins and are derived from leukocytes, lymphoblastoid and fibroblast cells during the activation of immune responses in humans and other vertebrate animals or by recombinant DNA methods. Interferon is classified into alfa, beta and gamma designations which correspond to leukocyte, fibroblast and type II interferons, respectively.

For the sake of clarity, the various kinds of interferon are simply referred to as "interferon" in the following description.

PCT Patent Application WO 88/03441 deals with interferon therapy and may be considered as a good reference text on interferon and its use. In this PCT Application, interferon may be used in contact with oral or pharyngeal mucosa, for instance in a lozenge as a solid unitary dosage adapted to be dissolved in saliva when placed in the mouth.

However, interferon which is commonly available in lyophilised form for application is highly hydrophobic and must be stabilised in order to be kept and used in homeopathic dosages. This is a key feature that seems to have been overlooked in the PCT Application cited above.

An object of the present invention is therefore to provide stabilised interferon compositions for homeopathic use and a method to stabilise interferon in order for this substance to be used in homeopathic dilutions.

Another object of the invention is to use arabic gum as a stabilising agent for stabilising interferon in homeopathic tablets. The method comprises adding arabic gum to an aqueous solution of lyophilised interferons.

Another object of the invention is a pre-stabilised interferon and a method to pre-stabilise interferon before homeophatic dilutions by using albumin. This method comprises adding albumin as a previous pre-stabilising agent to an aqueous solution of lyophilised interferons.

Another object of the invention is to use such stabilised interferon compositions in homeopathic dilutions in a tablet or a pastille or granules which will be made in contact with saliva in a patient's mouth in order to enter the oral submucous lymphatic plexi.

The present invention thus relates to a method of stabilising interferons in aqueous solution with arabic gum for the manufacture of sublingually administrated tablets in homeopathic concentrations.

In this case arabic gum will be used together with albumine as a pre-stabilisation agent and, in a preferred embodiment, various phosphates.

Such stabilised interferon composition may be kept in solution at room temperature without the use of refrigeration for up to 6 months to treat interferon sensitive viral infections and cancers.

Said compound has to be used once the homeopathic dilutions have been carried out, and can also be used earlier when preparing the base material which will be subjected to the homeophatic dilutions. In other words, this compound may be utilised twice successively during the manufacturing of the drug in its final form.

The invention also provides a formulation for stabilising interferon for dissolution in aqueous alcohol solution.

The invention further provides a method of applying the aqueous alcohol solution containing the aqueous alcohol solution containing interferon on a specially prepared excipient by homeopathic dilution procedure such as decimal homeopathic dilution procedure (DH) from 1x to 10x homeopathic concentrations (DH 1 to DH 10) for the manufacture of tablets.

This method of stabilisation can be applied to any kind of interferon like alfa, beta or gamma interferons derived from bovine and humans, or by recombinant DNA methods. Among interferons, human alfa interferon is preferred.

The invention relates also to a method for the preparation of a drug comprising a homeopathic amount of interferon composition, for instance from 1 I.U. to 2000 I.U. per 100 mg tablet, in which said tablet contains an excipient comprising arabic gum. Because of the homeopathic procedure, the above values of interferon from 1 I.U to 2000 I.U. are expressed in contents before dilutions.

This tablet may also contain lactose and starch and is used to stimulate the therapeutic response in human patients by making contact with saliva in the patient's mouth.

Interferon is a macromolecule which cannot pass through mucosae in general and mouth mucosa in particular. It will act by stimulating the immune system, by oral contact. Therefore, interferon contained in the tablets are likely not to enter the blood or lymphatic system of the patient. The tablets should preferably not be swallow as the interferon is likely to be destroyed in the stomach or the intestines and to become of no use.

This tablet can be administered sublingually for the prevention and treatment of various diseases such as hepatitis B+C, viral tumours and cancers, aids (HIV) and other viral infections sensitive to interferon treatment For example a tablet of 100 mg comprising between 100 to 300 I.U. (as expressed before homeopathic dilutions) of stabilised human alfa-interferon will be administrated one to four times a day.

The optimum results are obtained when the interferon used has been put into contact with albumin and already arabic gum.

For the foregoing, it is clear that arabic gum provides an adhesive surface for the interferon molecules.

The preparation of tablets containing interferon compositions in homeopathic dose may be divided in three steps. In a first step, a base material is prepared by dissolving lyophilised interferon in bidistilled water in the presence of lactose and by adding successively lyophilised albumin, sodium monohydrophosphate, sodium dihydrosulfate and arabic gum.

In a second step, this base material is subjected to a treatment of multidilutions as in the art in homeopathy, each time ten times (decimal dilutions) with an alcoholic aqueous solution. A typical treatment is fourth decimal treatment (DH 4).

In a third step, the alcoholic solution obtained is then sprayed on granules of excipient containing arabic gum, lactose and starch, and the mixture is vacuum dried and finally compressed into tablets.

A typical method of preparing tablets is given on the enclosed Table, which is an example of a homeopathic 4 DH dilution with corresponding typical weights, volumes and proportions to be used.

This example is given for illustrative purpose only. The clinical trial results of the invention are based on the application of human alfa-interferon in homeopathic concentration of 200 I.U. per 100 mg tablet, as expressed before dilution steps.

### Example 1

The invention is carried out in three stages which are as follows :

### I - Stabilisation of Interferon :

Base stock of 30 million I.U. of lyophilised high purity human alfa-interferon is thoroughly mixed with 20 ml of double distilled water, 20 g of lactose, 600 µg of lyophilised high purity (HP) albumin, 150 µg of sodium monohydrophosphate, 150 µg of sodium dihydrophosphate and 20 g of arabic gum to form the base material. The base material must be free of alcohol, and all ingredients are either of high purity or pharmaceutical grade.

### II - Dilution of Stabilised Human Alfa-Interferon by Homeopathic Decimal Method

The base material is then dissolved at room temperature in 10 times of the weight of 20% aqueous ethyl alcohol solution for the first dilution step and is further carried out in steps 10 times of the weight of 20% aqueous ethyl alcohol solution repeatedly to the fourth step to give the final solution. This operation is carried out under standard homeophatic procedure.

### III - Preparation of the Excipient and Application

The excipient is prepared by mixing thoroughly 3000 g of arabic gum (20%); 4500 g of lactose (30%); and 7500g of wheat or rice starch, all pharmaceutical grade which become granular in texture.

The final stock solution is then sprayed on the granules of the excipient with a regular spray nozzle used in homeopathic manufacture with sterilised, filtered air under one atmosphere pressure during less than 60 minutes while the entire mixture is being stirred by standard mixing procedures. Losses of stock solution vary between 5% and 50% depending on the equipment used. Tablets of 100 mg each containing 200 I.U. of stabilised human alfa-interferon are made by standard methods and vacuum dried at temperatures between 8ºC and 20ºC before packaging into blister packs.

The entire process must be carried out in strict conformity to the norms of homeopathy.

The tablets are to be used by patients sublingually to promote and enhance the immune response of the body for the treatment of viral infections sensitive to interferon.

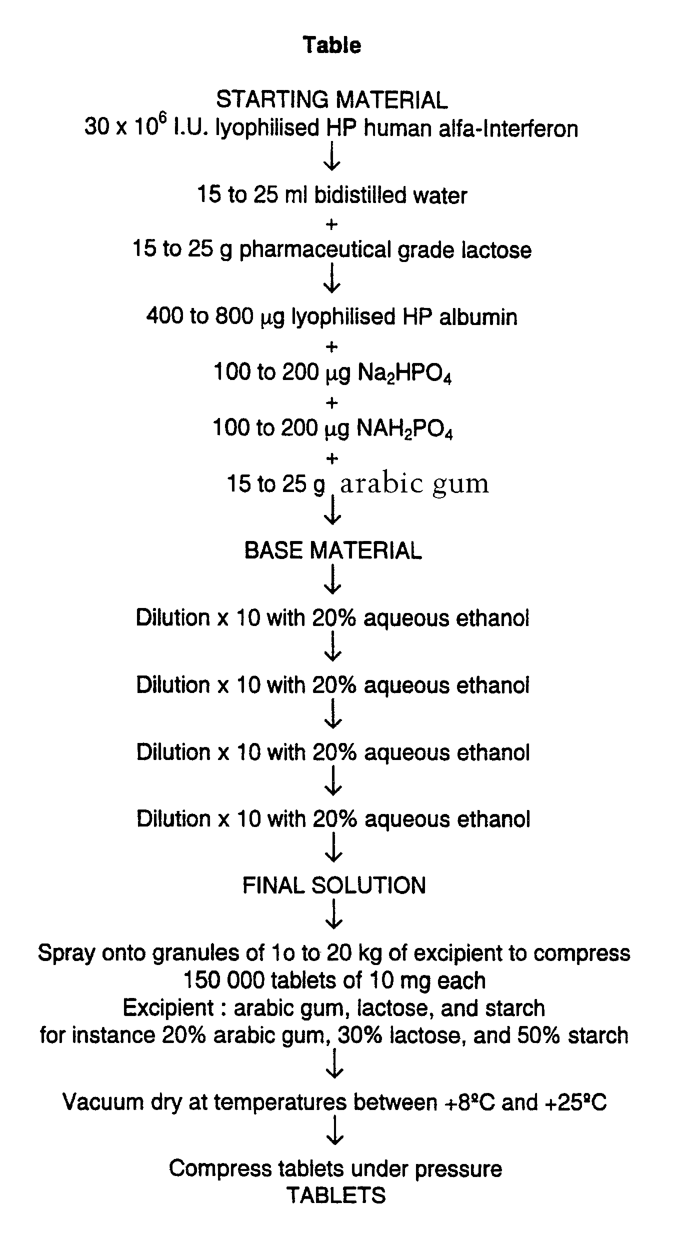

## Claims

1. Stabilised interferon compositions for sublingual administration in homeopathic concentrations, **characterised in that** the stabilising agent is arabic gum.

2. Stabilised interferon compositions according to claim 1, wherein interferons are alfa-, beta- or gamma-interferon derived from bovine and humans or by recombinant DNA methods, in particular human alfa-interferon.

3. Stabilised interferon compositions according to claims 1 or 2, comprising albumin as a pre-stabilising agent.

4. A method of stabilising interferons in aqueous solution which comprises adding arabic gum to an aqueous solution of lyophilised interferons.

5. A method according to claim 4, which comprises adding albumin as a previous pre-stabilising agent to an aqueous solution of lyophilised interferons.

6. A tablet comprising homeopathic amounts of stabilised interferons according to any of claims 1 to 3 and an excipient comprising arabic gum.

7. A tablet according to claim 6 containing homeopathic amounts of stabilised interferons from 1 I.U. to 2000 I.U. per 100 mg tablet and an excipient comprising arabic gum, lactose, and starch.

8. A tablet of 100 mg comprising 100 to 300 I.U. of stabilised human alfa-interferon according to any of claims 1 to 3 for sublingual administration one to four times a day for the prevention and treatment of hepatitis-B+C, viral induced tumours and cancers, HIV and other viral infections sensitive to interferon treatment.

9. A method for preparing tablets according to claim 8 which comprises diluting in homeopathic concentrations a stabilised interferons according to any of claims 1 to 3, spraying onto an excipient containing arabic gum, vacuum drying and compressing into tablets.

10. A method for preparing tablets according to claim 9 which comprises
a) adding successively albumin and arabic gum to an aqueous solution of lyophilised interferon,
b) diluting the aqueous solution obtained under standard homeopathic procedure in successive aqueous solutions containing ethanol to obtain a final solution, and
c) spraying this final solution onto an excipient containing arabic gum followed by vacuum drying and compressing into tablets.

## Patentansprüche

1. Stabilisierte Interferonzusammensetzungen zur sublingualen Verabreichung in homöopathischen Konzentrationen, **dadurch gekennzeichnet, dass** der Stabilisator Gummiarabikum ist.

2. Stabilisierte Interferonzusammensetzungen nach Anspruch 1, worin die Interferone vom Rind oder Menschen oder von Verfahren der rekombinanten DNA abgeleitetes Alpha-, Beta- oder Gamma-Interferon, insbesondere menschliches Alpha-Interferon, sind.

3. Stabilisierte Interferonzusammensetzungen nach Ansprüchen 1 oder 2, die Albumin als einen Vorstabilisator umfassen.

4. Verfahren, Interferone in wässriger Lösung zu stabilisieren, das ein Hinzufügen von Gummiarabikum zu einer wässrigen Lösung von gefriergetrockneten Interferonen umfasst.

5. Verfahren nach Anspruch 4, das umfasst, Albumin als vorherigen Vorstabilisator zu einer wässrigen Lösung gefriergetrockneter Interferone hinzuzufügen.

6. Tablette mit homöopathischen Mengen von stabilisierten Interferonen nach einem beliebigen der Ansprüche 1 bis 3 und einem Gummiarabikum umfassenden Arzneimittelträger.

7. Tablette nach Anspruch 6, homöopathische Mengen von stabilisierten Interferonen von 1 E. bis 2000 E. pro 100-mg-Tablette sowie einen Gummiarabikum, Lactose und Stärke umfassenden Arzneimittelträger enthaltend.

8. Eine 100 bis 300 E. von stabilisiertem menschlichem Alpha-Interferon nach einem beliebigen der Ansprüche 1 bis 3 enthaltende 100-mg-Tablette zur sublingualen Verabreichung ein- bis viermal täglich für die Verhütung und Behandlung von Hepatitis B+C, durch Viren induzierten Tumoren und Karzinomen, HIV und anderen, auf Interferonbehandlung ansprechenden Vireninfektionen.

9. Verfahren zur Herstellung von Tabletten nach Anspruch 8, das umfasst, ein stabilisierte Interferone nach einem beliebigen der Ansprüche 1 bis 3 zu homöopathischen Konzentrationen zu verdünnen, auf einen Gummiarabikum enthaltenden Arzneiträger aufzusprühen, unter Vakuum zu trocknen und zu Tabletten zu verpressen.

10. Verfahren, Tabletten nach Anspruch 9 herzustellen, das umfasst,
a) aufeinanderfolgend Albumin und Gummiarabikum zu einer wässrigen Lösung von gefriergetrocknetem Interferon hinzuzufügen,
b) die erhaltene wässrige Lösung nach homöopathischer Standardprozedur zu aufeinanderfolgenden, Ethanol enthaltenden wässrigen Lösungen zu verdünnen, um eine endgültige Lösung zu erhalten, und
c) diese endgültige Lösung auf einen Gummiarabikum enthaltenden Arzneiträger aufzusprühen, gefolgt von Trocknen unter Vakuum und Verpressen zu Tabletten.

## Revendications

1. Compositions stabilisées d'interféron pour administration sublinguale en concentrations homéopathiques, **caractérisées en ce que** l'agent de stabilisation est de la gomme arabique.

2. Compositions stabilisées d'interféron selon la revendication 1, dans lesquelles les interférons sont de l'alpha-, du beta- ou du gamma-interféron dérivés de bovins ou d'humains ou par des méthodes ADN recombinantes, en particulier de l'alpha-interféron humain.

3. Compositions stabilisées d'interféron selon les revendications 1 ou 2, comprenant de l'albumine comme agent de préstabilisation.

4. Méthode de stabilisation d'interférons en solution aqueuse qui comprend l'étape consistant à ajouter de la gomme arabique à une solution aqueuse d'interférons lyophilisés.

5. Méthode selon la revendication 4, qui comprend l'étape consistant à ajouter de l'albumine comme agent de préstabilisation préalable à une solution aqueuse d'interférons lyophilisés.

6. Tablette comprenant des quantités homéopathiques d'interférons stabilisés selon l'une des revendications 1 à 3 et un excipient comprenant de la gomme arabique.

7. Tablette selon la revendication 6, contenant des quantités homéopathiques d'interférons stabilisés de 1 U.I. à 2000 U.I. par 100 mg de tablette et un excipient comprenant de la gomme arabique, du lactose et de l'amidon.

8. Tablette de 100 mg comprenant de 100 à 300 U.I. d'alpha-interféron stabilisé selon l'une des revendications 1 à 3, pour administration sublinguale, une à quatre fois par jour pour la prévention et le traitement de l'hépatite B+C, des tumeurs et cancers induits par des virus, de VIH ou d'autres infections virales sensibles à un traitement à l'interféron.

9. Méthode pour préparer des tablettes selon la revendication 8, qui comprend les étapes consistant à diluer en concentrations homéopathiques un interféron stabilisé selon l'une des revendications 1 à 3, à atomiser sur un excipient contenant de la gomme arabique, à sécher sous vide et à comprimer en tablettes.

10. Méthode pour préparer des tablettes selon la revendication 9 qui comprend des étapes consistant à:
a) ajouter successivement de l'albumine et de la gomme arabique à une solution aqueuse d'interféron lyophilisé,
b) diluer la solution aqueuse obtenue selon des procédures standard de l'homéopathie en une solution aqueuse successive contenant de l'éthanol pour obtenir une solution finale, et
c) atomiser la solution finale sur un excipient contenant la gomme arabique, suivi par un séchage sous vide et une compression en tablettes.
